# EUROPEAN PATENT SPECIFICATION

(11) **EP 2 023 962 B1**
(45) Date of publication and mention of the grant of the patent: **11.09.2013**
(21) Application number: 07783878.7
(22) Date of filing: 17.05.2007
(51) Int. Cl.: C07K 14/47, C12N 1/15, C12N 1/21, C12N 5/10

(54) **Human oct-2 variant peptide chains, nucleic acids, and methods**
Peptidketten aus humaner Oct-2-variante, Nukleinsäuren und Verfahren
Chaînes peptidiques de variants de l'oct-2 humain, acides nucléiques, et procédés

(30) Priority: 17.05.2006 US 801127 P
(43) Date of publication of application: 18.02.2009
(73) Proprietor: Janssen Biotech, Inc., Horsham, PA 19044 (US)
(72) Inventor: DORAI, Haimanti, Exton, PA 19341 (US); LU, Jin, Boothwyn, PA 19061 (US)
(74) Representative: Marshall, Cameron John
(86) International application number: PCT/US2007/069151
(87) International publication number: WO 2007/137121

(56) References cited:
- WO-A2-2005/017121
- CLERC R G ET AL: "THE B CELL-SPECIFIC OCT-2 PROTEIN CONTAINS POU BOX AND HOMEO BOX-TYPE DOMAINS" GENES AND DEVELOPMENT, vol. 2, no. 12A, 1988, pages 1570-1581, XP002550670 ISSN: 0890-9369
- WIRTH T ET AL: "MULTIPLE OCT2 ISOFORMS ARE GENERATED BY ALTERNATIVE SPLICING" NUCLEIC ACIDS RESEARCH, vol. 19, no. 1, 1991, pages 43-52, XP002550671 ISSN: 0305-1048
- ZWILLING STEFAN ET AL: "The POU domains of the Oct1 and Oct2 transcription factors mediate specific interaction with TBP" NUCLEIC ACIDS RESEARCH, vol. 22, no. 9, 1994, pages 1655-1662, XP002550672 ISSN: 0305-1048
- SÁEZ ANA-ISABEL ET AL: "Analysis of octamer-binding transcription factors Oct2 and Oct1 and their coactivator BOB.1/OBF.1 in lymphomas." MODERN PATHOLOGY : AN OFFICIAL JOURNAL OF THE UNITED STATES AND CANADIAN ACADEMY OF PATHOLOGY, INC MAR 2002, vol. 15, no. 3, March 2002 (2002-03), pages 211-220, XP002550673 ISSN: 0893-3952
- CORCORAN L M ET AL: "Oct-2, although not required for early B-cell development, is critical for later B-cell maturation and for postnatal survival." GENES & DEVELOPMENT APR 1993, vol. 7, no. 4, April 1993 (1993-04), pages 570-582, XP002550674 ISSN: 0890-9369
- LIU YU-ZHEN ET AL: "Adjacent proline residues in the inhibitory domain of the Oct-2 transcription factor play distinct functional roles" NUCLEIC ACIDS RESEARCH, vol. 26, no. 10, 15 May 1998 (1998-05-15), pages 2464-2472, XP002550777 ISSN: 0305-1048
- CLERC: 'The B-cell-specific Oct-2 protein contains POU box- and homeo box-type domains' GENES & DEV. vol. 2, December 1988, pages 1570 - 1581, XP007102766
- LILLYCROP K A ET AL: "Alternative splicing of the Oct-2 transcription factor RNA is differentially regulated in neuronal cells and B cells and results in protein isoforms with opposite effects on the activity of octamer/TAATGARAT-containing promoters.", THE JOURNAL OF BIOLOGICAL CHEMISTRY 15 DEC 1992 LNKD- PUBMED:1281152, vol. 267, no. 35, 15 December 1992 (1992-12-15), pages 24960-24965, ISSN: 0021-9258
- CORCORAN LYNN M ET AL: "All known in vivo functions of the Oct-2 transcription factor require the C-terminal protein domain.", JOURNAL OF IMMUNOLOGY, vol. 172, no. 5, 1 March 2004 (2004-03-01) , pages 2962-2969, ISSN: 0022-1767
- ANNWEILER ARND ET AL: "Functional differences between the Oct2 transactivation domains determine the transactivation potential of individual Oct2 isoforms", NUCLEIC ACIDS RESEARCH, vol. 22, no. 20, 1994, pages 4250-4258, ISSN: 0305-1048

### SEQUENCE LISTING

<110> CENTOCOR, INC.
<120> HUMAN OCT-2 VARIANT PEPTIDE CHAINS, NUCLEIC ACIDS, AND METHODS
<130> CEN5136 PCT
<140> TO BE ASSIGNED
   <141> 2007-05-17
<150> 60/801, 127
   <151> 2006-05-17
<160> 19
<170> FastSEQ for Windows Version 4.0
<210> 1
   <211> 1389
   <212> DNA
   <213> Homo sapiens
<400> 1
   atggttcact ccagcatggg ggctccagaa ataagaatgt ctaagcccct ggaggccgag 60
   aagcaaggtc tggactcccc atcagagcac acagacaccg aaagaaatgg accagacact 120
   aatcatcaga acccccaaaa taagacctcc ccattctccg tgtccccaac tggccccagt 180
   acaaagatca aggctgaaga ccccagtggc gattcagccc cagcagcacc cctgccccct 240
   cagccggccc agcctcatct gccccaggcc caactcatgt tgacgggcag ccagctagct 300
   ggggacatac agcagctcct ccagctccag cagctggtgc ttgtgccagg ccaccacctc 360
   cagccacctg ctcagttcct gctaccgcag gcccagcaga gccagccagg cctgctaccg 420
   acaccaaatc tattccagct acctcagcaa acccagggag ctcttctgac ctcccagccc 480
   cgggccgggc ttcccacaca gccccccaaa tgcttggagc caccatccca ccccgaggag 540
   cccagtgatc tggaggagct ggagcaattc gcccgcacct tcaagcaacg ccgcatcaag 600
   ctgggcttca cgcagggtga tgtgggcctg gccatgggca agctctacgg caacgacttc 660
   agccagacga ccatttcccg cttcgaggcc ctcaacctga gcttcaagaa catgtgcaaa 720
   ctcaagcccc tcctggagaa gtggctcaac gatgcagaga ctatgtctgt ggactcaagc 780
   ctgcccagcc ccaaccagct gagcagcccc agcctgggtt tcgacggcct gcccggccgg 840
   agacgcaaga agaggaccag catcgagaca aacgtccgct tcgccttaga gaagagtttt 900
   ctagcgaacc agaagcctac ctcagaggag atcctgctga tcgccgagca gctgcacatg 960
   gagaaggaag tgatccgcgt ctggttctgc aaccggcgcc agaaggagaa acgcatcaac 1020
   ccctgcagtg cggcccccat gctgcccagc ccagggaagc cggccagcta cagcccccat 1080
   atggtcacac cccaaggggg cgcggggacc ttaccgttgt cccaagcttc cagcagtctg 1140
   agcacaacag ttactacctt atcctcagct gtggggacgc tccaccccag ccggacagct 1200
   ggagggggtg ggggcggggg cggggctgcg ccccccctca attccatccc ctctgtcact 1260
   cccccacccc cggccaccac caacagcaca aaccccagcc ctcaaggcag ccactcggct 1320
   atcggcttgt caggcctgaa ccccagcacg ggccctggcc tctggtggaa ccctgcccct 1380
   taccagcct                                                         1389
<210> 2
   <211> 463
   <212> PRT
   <213> Homo sapiens
<400> 2
   Met Val His Ser Ser Met Gly Ala Pro Glu Ile Arg Met Ser Lys Pro
    1               5                  10                  15
   Leu Glu Ala Glu Lys Gln Gly Leu Asp Ser Pro Ser Glu His Thr Asp
               20                  25                  30
   Thr Glu Arg Asn Gly Pro Asp Thr Asn His Gln Asn Pro Gln Asn Lys
           35                  40                  45
   Thr Ser Pro Phe Ser Val Ser Pro Thr Gly Pro Ser Thr Lys Ile Lys
       50                  55                  60
   Ala Glu Asp Pro Ser Gly Asp Ser Ala Pro Ala Ala Pro Leu Pro Pro
   65                  70                  75                  80
   Gln Pro Ala Gln Pro His Leu Pro Gln Ala Gln Leu Met Leu Thr Gly
                   85                  90                  95
   Ser Gln Leu Ala Gly Asp Ile Gln Gln Leu Leu Gln Leu Gln Gln Leu
               100                 105                 110
   Val Leu Val Pro Gly His His Leu Gln Pro Pro Ala Gln Phe Leu Leu
           115                 120                 125
   Pro Gln Ala Gln Gln Ser Gln Pro Gly Leu Leu Pro Thr Pro Asn Leu
       130                 135                 140
   Phe Gln Leu Pro Gln Gln Thr Gln Gly Ala Leu Leu Thr Ser Gln Pro
   145                 150                 155                 160
   Arg Ala Gly Leu Pro Thr Gln Pro Pro Lys Cys Leu Glu Pro Pro Ser
                   165                 170                 175
   His Pro Glu Glu Pro Ser Asp Leu Glu Glu Leu Glu Gln Phe Ala Arg
               180                 185                 190
   Thr Phe Lys Gln Arg Arg Ile Lys Leu Gly Phe Thr Gln Gly Asp Val
           195                 200                 205
   Gly Leu Ala Met Gly Lys Leu Tyr Gly Asn Asp Phe Ser Gln Thr Thr
       210                 215                 220
   Ile Ser Arg Phe Glu Ala Leu Asn Leu Ser Phe Lys Asn Met Cys Lys
   225                 230                 235                 240
   Leu Lys Pro Leu Leu Glu Lys Trp Leu Asn Asp Ala Glu Thr Met Ser
                   245                 250                 255
   Val Asp Ser Ser Leu Pro Ser Pro Asn Gln Leu Ser Ser Pro Ser Leu
               260                 265                 270
   Gly Phe Asp Gly Leu Pro Gly Arg Arg Arg Lys Lys Arg Thr Ser Ile
           275                 280                 285
   Glu Thr Asn Val Arg Phe Ala Leu Glu Lys Ser Phe Leu Ala Asn Gln
       290                 295                 300
   Lys Pro Thr Ser Glu Glu Ile Leu Leu Ile Ala Glu Gln Leu His Met
   305                 310                 315                 320
   Glu Lys Glu Val Ile Arg Val Trp Phe Cys Asn Arg Arg Gln Lys Glu
                   325                 330                 335
   Lys Arg Ile Asn Pro Cys Ser Ala Ala Pro Met Leu Pro Ser Pro Gly
               340                 345                 350
   Lys Pro Ala Ser Tyr Ser Pro His Met Val Thr Pro Gln Gly Gly Ala
           355                 360                 365
   Gly Thr Leu Pro Leu Ser Gln Ala Ser Ser Ser Leu Ser Thr Thr Val
       370                 375                 380
   Thr Thr Leu Ser Ser Ala Val Gly Thr Leu His Pro Ser Arg Thr Ala
   385                 390                 395                 400
   Gly Gly Gly Gly Gly Gly Gly Gly Ala Ala Pro Pro Leu Asn Ser Ile
                   405                 410                 415
   Pro Ser Val Thr Pro Pro Pro Pro Ala Thr Thr Asn Ser Thr Asn Pro
               420                 425                 430
   Ser Pro Gln Gly Ser His Ser Ala Ile Gly Leu Ser Gly Leu Asn Pro
           435                 440                 445
   Ser Thr Gly Pro Gly Leu Trp Trp Asn Pro Ala Pro Tyr Gln Pro
       450                 455                 460
<210> 3
   <211> 1353
   <212> DNA
   <213> Homo sapiens
<400> 3
   atggttcact ccagcatggg ggctccagaa ataagaatgt ctaagcccct ggaggccgag 60
   aagcaaggtc tggactcccc atcagagcac acagacaccg aaagaaatgg accagacact 120
   aatcatcaga acccccaaaa taagacctcc ccattctccg tgtccccaac tggccccagt 180
   acaaagatca aggctgaaga ccccagtggc gattcagccc cagcagcacc cctgccccct 240
   cagccggccc agcctcatct gccccaggcc caactcatgt tgacgggcag ccagctagct 300
   ggggacatac agcagctcct ccagctccag cagctggtgc ttgtgccagg ccaccacctc 360
   cagccacctg ctcagttcct gctaccgcag gcccagcaga gccagccagg cctgctaccg 420
   acaccaaatc tattccagct acctcagcaa acccagggag ctcttctgac ctcccagccc 480
   cgggccgggc ttcccacaca gccccccaaa tgcttggagc caccatccca ccccgaggag 540
   cccagtgatc tggaggagct ggagcaattc gcccgcacct tcaagcaacg ccgcatcaag 600
   ctgggcttca cgcagggtga tgtgggcctg gccatgggca agctctacgg caacgacttc 660
   agtcagacga ccatttcccg cttcgaggcc ctcaacctga gcttcaagaa catgtgcaaa 720
   ctcaagcccc tcctggagaa gtggctcaac gatgcagaga ctatgtctgt ggactcaagc 780
   ctgcccagcc ccaaccagct gagcagcccc agcctgggtt tcgacggcct gcccggccgg 840
   agacgcaaga agaggaccag catcgagaca aacgtccgct tcgccttaga gaagagtttt 900
   ctagcgaacc agaagcctac ctcagaggag atcctgctga tcgccgagca gctgcgcatg 960
   gagaaggaag tgatccgcgt ctggttctgc aaccggcgcc agaaggagaa acgcatcaac 1020
   ccctgcagtg cggcccccat gctgcccagc ccagggaagc cggccagcta cagcccccat 1080
   atggtcacac cccaaggggg cgcggggacc ttaccgttgt cccaagcttc cagcagtctg 1140
   agcacaacag ttactacctt atcctcagct gtggggacgc tccaccccag ccggacagct 1200
   ggagggggtg ggggcggggg cggggctgcg ccccccctca attccatccc ctctgtcact 1260
   cccccacccc cggccaccac caacagcaca aaccccagcc ctcaaggcag ccactcggct 1320
   atcggcttgt caggcctgaa ccccagcacg ggg                              1353
<210> 4
   <211> 451
   <212> PRT
   <213> Homo sapiens
<400> 4
   Met Val His Ser Ser Met Gly Ala Pro Glu Ile Arg Met Ser Lys Pro
    1               5                  10                  15
   Leu Glu Ala Glu Lys Gln Gly Leu Asp Ser Pro Ser Glu His Thr Asp
               20                  25                  30
   Thr Glu Arg Asn Gly Pro Asp Thr Asn His Gln Asn Pro Gln Asn Lys
           35                  40                  45
   Thr Ser Pro Phe Ser Val Ser Pro Thr Gly Pro Ser Thr Lys Ile Lys
       50                  55                  60
   Ala Glu Asp Pro Ser Gly Asp Ser Ala Pro Ala Ala Pro Leu Pro Pro
   65                  70                  75                  80
   Gln Pro Ala Gln Pro His Leu Pro Gln Ala Gln Leu Met Leu Thr Gly
                   85                  90                  95
   Ser Gln Leu Ala Gly Asp Ile Gln Gln Leu Leu Gln Leu Gln Gln Leu
               100                 105                 110
   Val Leu Val Pro Gly His His Leu Gln Pro Pro Ala Gln Phe Leu Leu
           115                 120                 125
   Pro Gln Ala Gln Gln Ser Gln Pro Gly Leu Leu Pro Thr Pro Asn Leu
       130                 135                 140
   Phe Gln Leu Pro Gln Gln Thr Gln Gly Ala Leu Leu Thr Ser Gln Pro
   145                 150                 155                 160
   Arg Ala Gly Leu Pro Thr Gln Pro Pro Lys Cys Leu Glu Pro Pro Ser
                   165                 170                 175
   His Pro Glu Glu Pro Ser Asp Leu Glu Glu Leu Glu Gln Phe Ala Arg
               180                 185                 190
   Thr Phe Lys Gln Arg Arg Ile Lys Leu Gly Phe Thr Gln Gly Asp Val
           195                 200                 205
   Gly Leu Ala Met Gly Lys Leu Tyr Gly Asn Asp Phe Ser Gln Thr Thr
       210                 215                 220
   Ile Ser Arg Phe Glu Ala Leu Asn Leu Ser Phe Lys Asn Met Cys Lys
   225                 230                 235                 240
   Leu Lys Pro Leu Leu Glu Lys Trp Leu Asn Asp Ala Glu Thr Met Ser
                   245                 250                 255
   Val Asp Ser Ser Leu Pro Ser Pro Asn Gln Leu Ser Ser Pro Ser Leu
               260                 265                 270
   Gly Phe Asp Gly Leu Pro Gly Arg Arg Arg Lys Lys Arg Thr Ser Ile
           275                 280                 285
   Glu Thr Asn Val Arg Phe Ala Leu Glu Lys Ser Phe Leu Ala Asn Gln
       290                 295                 300
   Lys Pro Thr Ser Glu Glu Ile Leu Leu Ile Ala Glu Gln Leu Arg Met
   305                 310                 315                 320
   Glu Lys Glu Val Ile Arg Val Trp Phe Cys Asn Arg Arg Gln Lys Glu
                   325                 330                 335
   Lys Arg Ile Asn Pro Cys Ser Ala Ala Pro Met Leu Pro Ser Pro Gly
               340                 345                 350
   Lys Pro Ala Ser Tyr Ser Pro His Met Val Thr Pro Gln Gly Gly Ala
           355                 360                 365
   Gly Thr Leu Pro Leu Ser Gln Ala Ser Ser Ser Leu Ser Thr Thr Val
       370                 375                 380
   Thr Thr Leu Ser Ser Ala Val Gly Thr Leu His Pro Ser Arg Thr Ala
   385                 390                 395                 400
   Gly Gly Gly Gly Gly Gly Gly Gly Ala Ala Pro Pro Leu Asn Ser Ile
                   405                 410                 415
   Pro Ser Val Thr Pro Pro Pro Pro Ala Thr Thr Asn Ser Thr Asn Pro
               420                 425                 430
   Ser Pro Gln Gly Ser His Ser Ala Ile Gly Leu Ser Gly Leu Asn Pro
           435                 440                 445
   Ser Thr Gly
       450
<210> 5
   <211> 1353
   <212> DNA
   <213> Homo sapiens
<400> 5
   atggttcact ccagcatggg ggctccagaa ataagaatgt ctaagcccct ggaggccgag 60
   aagcaaggtc tggactcccc atcagagcac acagacaccg aaagaaatgg accagacact 120
   aatcatcaga acccccaaaa taagacctcc ccattctccg tgtccccaac tggccccagt 180
   acaaagatca aggctgaaga ccccagtggc gattcagccc cagcagcacc cctgccccct 240
   cagccggccc agcctcatct gccccaggcc caactcatgt tgacgggcag ccagctagct 300
   ggggacatac agcagctcct ccagctccag cagctggtgc ttgtgtcagg ccaccacctc 360
   cagccacctg ctcagttcct gctaccgcag gcccagcaga gccagccagg cctgctaccg 420
   acaccaaatc tattccagct acctcagcaa acccagggag ctcttctgac ctcccagccc 480
   cgggccgggc ttcccacaca gccccccaaa tgcttggagc caccatccca ccccgaggag 540
   cccagtgatc tggaggagct ggagcaattc gcccgcacct tcaagcaacg ccgcatcaag 600
   ctgggcttca cgcagggtga tgtgggcctg gccatgggca agctctacgg caacgacttc 660
   agtcagacga ccatttcccg cttcgaggcc ctcaacctga gcttcaagaa catgtgcaaa 720
   ctcaagcccc tcctggagaa gtggctcaac gatgcagaga ctatgtctgt ggactcaagc 780
   ctgcccagcc ccaaccagct gagcagcccc agcctgggtt tcgacggcct gcccggccgg 840
   agacgcaaga agaggaccag catcgagaca aacgtccgct tcgccttaga gaagagtttt 900
   ctagcgaacc agaagcctac ctcagaggag atcctgctga tcgccgagca gctgcgcatg 960
   gagaaggaag tgatccgcgt ctggttctgc aaccggcgcc agaaggagaa acgcatcaac 1020
   ccctgcagtg cggcccccat gctgcccagc ccagggaagc cggccagcta cagcccccat 1080
   atggtcacac cccaaggggg cgcggggacc ttaccgttgt cccaagcttc cagcagtctg 1140
   agcacaacag ttactacctt atcctcagct gtggggacgc tccaccccag ccggacagct 1200
   ggagggggtg ggggcggggg cggggctgcg ccccccctca attccatccc ctctgtcact 1260
   cccccacccc cggccaccac caacagcaca aaccccagcc ctcaaggcag ccactcggct 1320
   atcggcttgt caggcctgaa ccccagcacg ggg                              1353
<210> 6
   <211> 451
   <212> PRT
   <213> Homo sapiens
<400> 6
   Met Val His Ser Ser Met Gly Ala Pro Glu Ile Arg Met Ser Lys Pro
    1               5                  10                  15
   Leu Glu Ala Glu Lys Gln Gly Leu Asp Ser Pro Ser Glu His Thr Asp
               20                  25                  30
   Thr Glu Arg Asn Gly Pro Asp Thr Asn His Gln Asn Pro Gln Asn Lys
           35                  40                  45
   Thr Ser Pro Phe Ser Val Ser Pro Thr Gly Pro Ser Thr Lys Ile Lys
       50                  55                  60
   Ala Glu Asp Pro Ser Gly Asp Ser Ala Pro Ala Ala Pro Leu Pro Pro
   65                  70                  75                  80
   Gln Pro Ala Gln Pro His Leu Pro Gln Ala Gln Leu Met Leu Thr Gly
                   85                  90                  95
   Ser Gln Leu Ala Gly Asp Ile Gln Gln Leu Leu Gln Leu Gln Gln Leu
               100                 105                 110
   Val Leu Val Ser Gly His His Leu Gln Pro Pro Ala Gln Phe Leu Leu
           115                 120                 125
   Pro Gln Ala Gln Gln Ser Gln Pro Gly Leu Leu Pro Thr Pro Asn Leu
       130                 135                 140
   Phe Gln Leu Pro Gln Gln Thr Gln Gly Ala Leu Leu Thr Ser Gln Pro
   145                 150                 155                 160
   Arg Ala Gly Leu Pro Thr Gln Pro Pro Lys Cys Leu Glu Pro Pro Ser
                   165                 170                 175
   His Pro Glu Glu Pro Ser Asp Leu Glu Glu Leu Glu Gln Phe Ala Arg
               180                 185                 190
   Thr Phe Lys Gln Arg Arg Ile Lys Leu Gly Phe Thr Gln Gly Asp Val
           195                 200                 205
   Gly Leu Ala Met Gly Lys Leu Tyr Gly Asn Asp Phe Ser Gln Thr Thr
       210                 215                 220
   Ile Ser Arg Phe Glu Ala Leu Asn Leu Ser Phe Lys Asn Met Cys Lys
   225                 230                 235                 240
   Leu Lys Pro Leu Leu Glu Lys Trp Leu Asn Asp Ala Glu Thr Met Ser
                   245                 250                 255
   Val Asp Ser Ser Leu Pro Ser Pro Asn Gln Leu Ser Ser Pro Ser Leu
               260                 265                 270
   Gly Phe Asp Gly Leu Pro Gly Arg Arg Arg Lys Lys Arg Thr Ser Ile
           275                 280                 285
   Glu Thr Asn Val Arg Phe Ala Leu Glu Lys Ser Phe Leu Ala Asn Gln
       290                 295                 300
   Lys Pro Thr Ser Glu Glu Ile Leu Leu Ile Ala Glu Gln Leu Arg Met
   305                 310                 315                 320
   Glu Lys Glu Val Ile Arg Val Trp Phe Cys Asn Arg Arg Gln Lys Glu
                   325                 330                 335
   Lys Arg Ile Asn Pro Cys Ser Ala Ala Pro Met Leu Pro Ser Pro Gly
               340                 345                 350
   Lys Pro Ala Ser Tyr Ser Pro His Met Val Thr Pro Gln Gly Gly Ala
           355                 360                 365
   Gly Thr Leu Pro Leu Ser Gln Ala Ser Ser Ser Leu Ser Thr Thr Val
       370                 375                 380
   Thr Thr Leu Ser Ser Ala Val Gly Thr Leu His Pro Ser Arg Thr Ala
   385                 390                 395                 400
   Gly Gly Gly Gly Gly Gly Gly Gly Ala Ala Pro Pro Leu Asn Ser Ile
                   405                 410                 415
   Pro Ser Val Thr Pro Pro Pro Pro Ala Thr Thr Asn Ser Thr Asn Pro
               420                 425                 430
   Ser Pro Gln Gly Ser His Ser Ala Ile Gly Leu Ser Gly Leu Asn Pro
           435                 440                 445
   Ser Thr Gly
       450
<210> 7
   <211> 768
   <212> DNA
   <213> Homo sapiens
<400> 7
   atgctctggc aaaaacccac agctccggag caagccccag ccccggcccg gccataccag 60
   ggcgtccgtg tgaaggagcc agtgaaggaa ctgctgagga ggaagcgagg ccacgccagc 120
   agtggggcag cacctgcacc tacggcggtg gtgctgcccc atcagcccct ggcgacctac 180
   accacagtgg gtccttcctg cctggacatg gaaggttctg tgtctgcagt gacagaggag 240
   gctgccctgt gtgccggctg gctctcccag cccaccccgg ccaccctgca gcccctggcc 300
   ccatggacac cttacaccga gtatgtgccc catgaagctg tcagctgccc ctactcagct 360
   gacatgtatg tgcagcccgt gtgccccagc tacacggtgg tggggccctc ctcagtgttg 420
   gcctatgcct ctccgccact catcaccaat gtcacgacaa gaagctccgc cacgcccgca 480
   gtggggcccc cgctggaggg cccagagcac caggcacccc tcacctattt cccgtggcct 540
   cagccccttt ccacactacc cacctccacc ctgcagtacc ggcctccggc cccagcccta 600
   cctgggcccc agtttgtcca gctccccatc tctatcccag agccagtcct tcaggacatg 660
   gaagacccca gaagagccgc cagctcgttg accatcgaca agctgctttt ggaggaagag 720
   gatagcgacg cctatgcgct taaccacact ctctctgtgg aaggcttt             768
<210> 8
   <211> 256
   <212> PRT
   <213> Homo sapiens
<400> 8
   Met Leu Trp Gln Lys Pro Thr Ala Pro Glu Gln Ala Pro Ala Pro Ala
   1               5                   10                  15
   Arg Pro Tyr Gln Gly Val Arg Val Lys Glu Pro Val Lys Glu Leu Leu
               20                  25                  30
   Arg Arg Lys Arg Gly His Ala Ser Ser Gly Ala Ala Pro Ala Pro Thr
           35                  40                  45
   Ala Val Val Leu Pro His Gln Pro Leu Ala Thr Tyr Thr Thr Val Gly
       50                  55                  60
   Pro Ser Cys Leu Asp Met Glu Gly Ser Val Ser Ala Val Thr Glu Glu
   65                  70                  75                  80
   Ala Ala Leu Cys Ala Gly Trp Leu Ser Gln Pro Thr Pro Ala Thr Leu
                   85                  90                  95
   Gln Pro Leu Ala Pro Trp Thr Pro Tyr Thr Glu Tyr Val Pro His Glu
               100                 105                 110
   Ala Val Ser Cys Pro Tyr Ser Ala Asp Met Tyr Val Gln Pro Val Cys
           115                 120                 125
   Pro Ser Tyr Thr Val Val Gly Pro Ser Ser Val Leu Ala Tyr Ala Ser
       130                 135                 140
   Pro Pro Leu Ile Thr Asn Val Thr Thr Arg Ser Ser Ala Thr Pro Ala
   145                 150                 155                 160
   Val Gly Pro Pro Leu Glu Gly Pro Glu His Gln Ala Pro Leu Thr Tyr
                   165                 170                 175
   Phe Pro Trp Pro Gln Pro Leu Ser Thr Leu Pro Thr Ser Thr Leu Gln
               180                 185                 190
   Tyr Arg Pro Pro Ala Pro Ala Leu Pro Gly Pro Gln Phe Val Gln Leu
           195                 200                 205
   Pro Ile Ser Ile Pro Glu Pro Val Leu Gln Asp Met Glu Asp Pro Arg
       210                 215                 220
   Arg Ala Ala Ser Ser Leu Thr Ile Asp Lys Leu Leu Leu Glu Glu Glu
   225                 230                 235                 240
   Asp Ser Asp Ala Tyr Ala Leu Asn His Thr Leu Ser Val Glu Gly Phe
                   245                 250                 255
<210> 9
   <211> 768
   <212> DNA
   <213> Mus musculus
<400> 9
   atgctctggc aaaaatccac agctccagag caagctcctg ccccaccaag gccataccag 60
   ggtgttcgag tcaaggagcc agtgaaggag ctactgagaa gaaagcgtgg ccataccagc 120
   gttggggcag ctgggccacc gaccgcggtg gtactgcccc accagcccct ggccacctac 180
   agcactgtgg gtccttcctg ccttgacatg gaggtttctg cttccacagt gacagaggag 240
   ggaacattat gtgctggctg gctctcccaa cctgccccgg ccactcttca gccattggct 300
   ccatggacac cctacacgga gtatgtgtcc catgaagctg tcagctgccc ctactccact 360
   gacatgtacg tgcagcctgt gtgccccagc tacacagtgg tgggaccctc ctcggtgttg 420
   acctatgctt ctccaccact catcactaat gtcacgccaa gaagcactgc tacacccgcg 480
   gtggggcccc agctggaggg tcccgagcac caggcgcccc tcacttattt cccgtggcct 540
   cagccccttt ccacactgcc cacctccagc ctgcagtatc aacctcctgc cccaaccctg 600
   tctgggcccc agtttgtcca gctccccatc tctatcccag agccagtcct tcaggacatg 660
   gatgacccca gaagggccat cagctccctg accattgaca agctgcttct ggaggaagag 720
   gaaagcaaca cgtacgagct caaccacacc ctctccgtgg agggcttt             768
<210> 10
   <211> 256
   <212> PRT
   <213> Mus musculus
<400> 10
   Met Leu Trp Gln Lys Ser Thr Ala Pro Glu Gln Ala Pro Ala Pro Pro
   1               5                   10                  15
   Arg Pro Tyr Gln Gly Val Arg Val Lys Glu Pro Val Lys Glu Leu Leu
               20                  25                  30
   Arg Arg Lys Arg Gly His Thr Ser Val Gly Ala Ala Gly Pro Pro Thr
           35                  40                  45
   Ala Val Val Leu Pro His Gln Pro Leu Ala Thr Tyr Ser Thr Val Gly
       50                  55                  60
   Pro Ser Cys Leu Asp Met Glu Val Ser Ala Ser Thr Val Thr Glu Glu
   65                  70                  75                  80
   Gly Thr Leu Cys Ala Gly Trp Leu Ser Gln Pro Ala Pro Ala Thr Leu
                   85                  90                  95
   Gln Pro Leu Ala Pro Trp Thr Pro Tyr Thr Glu Tyr Val Ser His Glu
               100                 105                 110
   Ala Val Ser Cys Pro Tyr Ser Thr Asp Met Tyr Val Gln Pro Val Cys
           115                 120                 125
   Pro Ser Tyr Thr Val Val Gly Pro Ser Ser Val Leu Thr Tyr Ala Ser
       130                 135                 140
   Pro Pro Leu Ile Thr Asn Val Thr Pro Arg Ser Thr Ala Thr Pro Ala
   145                 150                 155                 160
   Val Gly Pro Gln Leu Glu Gly Pro Glu His Gln Ala Pro Leu Thr Tyr
                   165                 170                 175
   Phe Pro Trp Pro Gln Pro Leu Ser Thr Leu Pro Thr Ser Ser Leu Gln
               180                 185                 190
   Tyr Gln Pro Pro Ala Pro Thr Leu Ser Gly Pro Gln Phe Val Gln Leu
           195                 200                 205
   Pro Ile Ser Ile Pro Glu Pro Val Leu Gln Asp Met Asp Asp Pro Arg
       210                 215                 220
   Arg Ala Ile Ser Ser Leu Thr Ile Asp Lys Leu Leu Leu Glu Glu Glu
   225                 230                 235                 240
   Glu Ser Asn Thr Tyr Glu Leu Asn His Thr Leu Ser Val Glu Gly Phe
                   245                 250                 255
<210> 11
   <211> 17
   <212> DNA
   <213> Artificial Sequence
<220>
   <223> Forward primer for cloning nucleic acids encoding Homo sapiens OCT-2 variant peptide chains.
<400> 11
   ggcagcatgg ttcactc 17
<210> 12
   <211> 16
   <212> DNA
   <213> Artificial Sequence
<220>
   <223> Reverse primer for cloning nucleic acids encoding Homo sapiens OCT-2 variant peptide chains.
<400> 12
   gtgcacccac ttaccc 16
<210> 13
   <211> 1401
   <212> DNA
   <213> Homo sapiens
<400> 13
   atggttcact ccagcatggg ggctccagaa ataagaatgt ctaagcccct ggaggccgag 60
   aagcaaggtc tggactcccc atcagagcac acagacaccg aaagaaatgg accagacact 120
   aatcatcaga acccccaaaa taagacctcc ccattctccg tgtccccaac tggccccagt 180
   acaaagatca aggctgaaga ccccagtggc gattcagccc cagcagcacc cctgccccct 240
   cagccggccc agcctcatct gccccaggcc caactcatgt tgacgggcag ccagctagct 300
   ggggacatac agcagctcct ccagctccag cagctggtgc ttgtgccagg ccaccacctc 360
   cagccacctg ctcagttcct gctaccgcag gcccagcaga gccagccagg cctgctaccg 420
   acaccaaatc tattccagct acctcagcaa acccagggag ctcttctgac ctcccagccc 480
   cgggccgggc ttcccacaca ggccgtgacc cgccctacgc tgcccgaccc gcacctctcg 540
   cacccgcagc cccccaaatg cttggagcca ccatcccacc ccgaggagcc cagtgatctg 600
   gaggagctgg agcaattcgc ccgcaccttc aagcaacgcc gcatcaagct gggcttcacg 660
   cagggtgatg tgggcctggc catgggcaag ctctacggca acgacttcag ccagacgacc 720
   atttcccgct tcgaggccct caacctgagc ttcaagaaca tgtgcaaact caagcccctc 780
   ctggagaagt ggctcaacga tgcagagact atgtctgtgg actcaagcct gcccagcccc 840
   aaccagctga gcagccccag cctgggtttc gacggcctgc ccggccggag acgcaagaag 900
   aggaccagca tcgagacaaa cgtccgcttc gccttagaga agagttttct agcgaaccag 960
   aagcctacct cagaggagat cctgctgatc gccgagcagc tgcacatgga gaaggaagtg 1020
   atccgcgtct ggttctgcaa ccggcgccag aaggagaaac gcatcaaccc ctgcagtgcg 1080
   gcccccatgc tgcccagccc agggaagccg gccagctaca gcccccatat ggtcacaccc 1140
   caagggggcg cggggacctt accgttgtcc caagcttcca gcagtctgag cacaacagtt 1200
   actaccttat cctcagctgt ggggacgctc caccccagcc ggacagctgg agggggtggg 1260
   ggcgggggcg gggctgcgcc ccccctcaat tccatcccct ctgtcactcc cccacccccg 1320
   gccaccacca acagcacaaa ccccagccct caaggcagcc actcggctat cggcttgtca 1380
   ggcctgaacc ccagcacggg g                                           1401
<210> 14
   <211> 467
   <212> PRT
   <213> Homo sapiens
<400> 14
   Met Val His Ser Ser Met Gly Ala Pro Glu Ile Arg Met Ser Lys Pro
    1               5                  10                  15
   Leu Glu Ala Glu Lys Gln Gly Leu Asp Ser Pro Ser Glu His Thr Asp
               20                  25                  30
   Thr Glu Arg Asn Gly Pro Asp Thr Asn His Gln Asn Pro Gln Asn Lys
           35                  40                  45
   Thr Ser Pro Phe Ser Val Ser Pro Thr Gly Pro Ser Thr Lys Ile Lys
       50                  55                  60
   Ala Glu Asp Pro Ser Gly Asp Ser Ala Pro Ala Ala Pro Leu Pro Pro
   65                  70                  75                  80
   Gln Pro Ala Gln Pro His Leu Pro Gln Ala Gln Leu Met Leu Thr Gly
                   85                  90                  95
   Ser Gln Leu Ala Gly Asp Ile Gln Gln Leu Leu Gln Leu Gln Gln Leu
               100                 105                 110
   Val Leu Val Pro Gly His His Leu Gln Pro Pro Ala Gln Phe Leu Leu
           115                 120                 125
   Pro Gln Ala Gln Gln Ser Gln Pro Gly Leu Leu Pro Thr Pro Asn Leu
       130                 135                 140
   Phe Gln Leu Pro Gln Gln Thr Gln Gly Ala Leu Leu Thr Ser Gln Pro
   145                 150                 155                 160
   Arg Ala Gly Leu Pro Thr Gln Ala Val Thr Arg Pro Thr Leu Pro Asp
                   165                 170                 175
   Pro His Leu Ser His Pro Gln Pro Pro Lys Cys Leu Glu Pro Pro Ser
               180                 185                 190
   His Pro Glu Glu Pro Ser Asp Leu Glu Glu Leu Glu Gln Phe Ala Arg
           195                 200                 205
   Thr Phe Lys Gln Arg Arg Ile Lys Leu Gly Phe Thr Gln Gly Asp Val
       210                 215                 220
   Gly Leu Ala Met Gly Lys Leu Tyr Gly Asn Asp Phe Ser Gln Thr Thr
   225                 230                 235                 240
   Ile Ser Arg Phe Glu Ala Leu Asn Leu Ser Phe Lys Asn Met Cys Lys
                   245                 250                 255
   Leu Lys Pro Leu Leu Glu Lys Trp Leu Asn Asp Ala Glu Thr Met Ser
               260                 265                 270
   Val Asp Ser Ser Leu Pro Ser Pro Asn Gln Leu Ser Ser Pro Ser Leu
           275                 280                 285
   Gly Phe Asp Gly Leu Pro Gly Arg Arg Arg Lys Lys Arg Thr Ser Ile
       290                 295                 300
   Glu Thr Asn Val Arg Phe Ala Leu Glu Lys Ser Phe Leu Ala Asn Gln
   305                 310                 315                 320
   Lys Pro Thr Ser Glu Glu Ile Leu Leu Ile Ala Glu Gln Leu His Met
                   325                 330                 335
   Glu Lys Glu Val Ile Arg Val Trp Phe Cys Asn Arg Arg Gln Lys Glu
               340                 345                 350
   Lys Arg Ile Asn Pro Cys Ser Ala Ala Pro Met Leu Pro Ser Pro Gly
           355                 360                 365
   Lys Pro Ala Ser Tyr Ser Pro His Met Val Thr Pro Gln Gly Gly Ala
       370                 375                 380
   Gly Thr Leu Pro Leu Ser Gln Ala Ser Ser Ser Leu Ser Thr Thr Val
   385                 390                 395                 400
   Thr Thr Leu Ser Ser Ala Val Gly Thr Leu His Pro Ser Arg Thr Ala
                   405                 410                 415
   Gly Gly Gly Gly Gly Gly Gly Gly Ala Ala Pro Pro Leu Asn Ser Ile
               420                 425                 430
   Pro Ser Val Thr Pro Pro Pro Pro Ala Thr Thr Asn Ser Thr Asn Pro
           435                 440                 445
   Ser Pro Gln Gly Ser His Ser Ala Ile Gly Leu Ser Gly Leu Asn Pro
       450                 455                 460
   Ser Thr Gly
   465
<210> 15
   <211> 10
   <212> DNA
   <213> Artificial Sequence
<220>
   <221> unsure
   <222> (2)
   <223> Consensus "octamer" DNA sequence bound by OCT-2 peptide chains. N is any nucleic acid.
<400> 15
   tnatttgcat 10
<210> 16
   <211> 5
   <212> PRT
   <213> Artificial Sequence
<220>
   <223> Five carboxy terminal residues of OCT-2 variant peptide chain.
<400> 16
   Asn Pro Ser Thr Gly
   1               5
<210> 17
   <211> 5
   <212> PRT
   <213> Artificial Sequence
<220>
   <223> Five carboxy terminal residues of OCT-2 variant peptide chain.
<400> 17
   Asn Pro Ser Ala Gly
   1               5
<210> 18
   <211> 5
   <212> PRT
   <213> Artificial Sequence
<220>
   <221> unsure
   <222> (4)
   <223> Five carboxy terminal residues of OCT-2 variant peptide chain where Xaa can be any one of the twenty naturally occurring amino acids
<400> 18
   Asn Pro Ser Xaa Gly
    1               5
<210> 19
   <211> 12
   <212> PRT
   <213> Artificial Sequence
<220>
   <223> Twelve carboxy terminal residues of Homo sapiens wild-type/archetypal OCT-2 peptide chain.
<400> 19
   Pro Gly Leu Trp Trp Asn Pro Ala Pro Tyr Gln Pro
    1               5                   10

## Description

### Field of the Invention

The present invention relates to *Homo sapiens* OCT-2 variant peptide chains, polynucleotides encoding these peptide chains, cells comprising these polynucleotides, and methods of using the forgoing.

### Background of the Invention

Large-scale commercial production of proteins, such as antibodies, typically relies on expression of the protein by cultured eukaryotic cells. In general, it is recognized in the art that increasing mRNA copy number results in increased protein expression. Additionally, bioactive RNAs such as silencing RNAs (siRNA) are useful to prevent the expression of genes that produce undesired effects in cells. Large-scale production of proteins by cultured eukaryotic cells and bioactive RNA technologies are both dependent on the efficient transcription of genes into RNA encoding proteins or bioactive RNAs, respectively. However, low protein expression or low RNA transcript levels are common problems encountered in the use of these technologies.

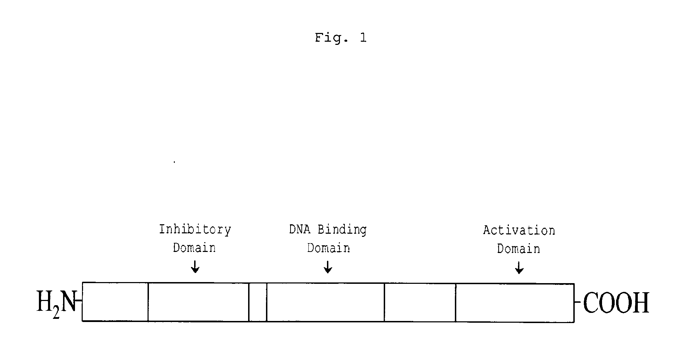

The OCT-2 protein and its known homologs are transcription factors capable of increasing the production of RNA transcripts from genes responsive to this protein. The predominant, bioactive form of *Homo sapiens* OCT-2 (SEQ ID NO: 2) consists of 463 amino acid residues and contains an inhibitory domain, a DNA binding domain, and an activation domain (Fig. 1) Splice variants of Oct-2 are known in human and mouse. (see Clerc et al., Genes and Development, 2 : 1570 (1988) and Wirth et al., Nucleic acids research 19:43 (1991). The DNA binding domain of the OCT-2 protein binds the "octamer site" having the consensus sequence 5'-TNATTTGCAT-3' (SEQ ID NO: 15; where N is any nucleic acid residue) in the promoter or regulatory region of OCT-2 responsive genes. *See* Müller et al. in Nature 336: 544 (1988). After DNA binding, the activation domain of the OCT-2 protein is believed to interact with the *Homo sapiens* co-activator protein OBF-1 (SEQ ID NO: 8) to stabilize formation of an active RNA polymerase II protein complex that can produce RNA transcripts. *See* Boss, Current Opin. In Immunol. 9: 107 (1997). The activity of OCT-2 increases the rate of formation of active RNA polymerase II protein complexes resulting in an increase in the production of RNA transcripts from OCT-2 responsive genes. In Corcoran et al., Journal of Immunology, 172 : 2962 (2004) the C-terminal activation domain is reported to be essential for Oct-2 function in vivo.

Genes may be naturally OCT-2 responsive or engineered to become OCT-2 responsive by inserting an "octamer" DNA sequence into the promoter or regulatory region of the gene. Consequently, it is expected that low levels of protein expression or low bioactive RNA levels could be increased by overexpressing OCT-2 alone or with the OBF-1 coactivator protein to increase transcription of OCT-2 responsive genes. Thus, a need exists for novel OCT-2 compositions and effective methods for increasing the expression or transcription of OCT-2 responsive genes.

### Brief Description of the Drawings

Fig. 1. Functional domains of a *Homo sapiens* OCT-2 peptide chain (SEQ ID NO: 2). Drawing is not to scale.

Fig. 2. Multiple sequence alignment analysis of the wild-type, archetypical *Homo sapiens* OCT-2 protein (NP_002689; SEQ ID NO: 2); the *Homo sapiens* OCT-2 Clone #19 Variant Protein (Clone #19; SEQ ID NO: 6); the *Homo sapiens* OCT-2 Clone #38 Variant Protein (Clone#38; SEQ ID NO: 4); and the *Homo sapiens* OCT-2 protein predicted to be encoded by coding sequence 1 (CDS 1) of Accession M36653 (SEQ ID NO: 14).

Fig. 3. Overexpression of *Homo sapiens* OCT-2 Clone #38 Variant Protein (SEQ ID NO: 4) alone and in combination with the *Mus musculus* OBF-1 protein (SEQ ID NO: 10) increases OCT-2 responsive antibody heavy and light chain gene transcript levels in eukaryotic C463A cells.

Fig. 4. Overexpression of *Homo sapiens* OCT-2 Clone #38 Variant Protein (SEQ ID NO: 4) alone and in combination with the *Mus musculus* OBF-1 protein (SEQ ID NO: 10) increases OCT-2 responsive antibody gene expression levels in eukaryotic C463A cells.

### Summary of the Invention

In a first aspect, the invention provides an expression vector comprising a nucleic acid encoding a peptide chain comprising an amino acid sequence wherein the nucleic acid encodes a peptide chain having:
(i) the amino acid sequence shown in SEQ ID NO: 4; or
(ii) the amino acid sequence shown in SEQ ID NO: 6.

In a second aspect, the invention provides an isolated peptide chain comprising an amino acid sequence, wherein the peptide chain has:
(i) the amino acid sequence shown a SEQ ID NO: 4; or
(ii) the amino acid sequence shown in SEQ ID NO: 6.

Another aspect of the invention is an in vitro method of increasing the expression of an OCT-2 responsive gene by a eukaryotic cell comprising the steps of providing a eukaryotic cell comprising an OCT-2 responsive gene; providing the eukaryotic cell with a first nucleic acid comprising a nucleic acid encoding a first peptide chain having an amino acid sequence with at least 90% identity to amino acid residues 1 to 447 of SEQ ID NO: 6, wherein the five amino acid residues at the carboxy terminus of the first peptide chain comprise the amino acid sequence Asparagine-Proline-Serine-Xaa-Glycine (SEQ ID NO: 18) where Xaa is any L-amino acid; and expressing the first peptide chain in the eukaryotic cell whereby the expression of the OCT-2 responsive gene is increased relative to a control eukaryotic cell that was not provided with the first nucleic acid.

Another aspect of the invention is an in vitro method of increasing the expression of an OCT-2 responsive gene by a eukaryotic cell comprising the steps of providing a eukaryotic cell comprising an OCT-2 responsive gene; providing the eukaryotic cell with a first nucleic acid comprising a nucleic acid encoding a first peptide chain comprising an amino acid sequence with at least 90% identity to amino acid residues 1 to 447 of SEQ ID NO: 6, wherein the five amino acid residues at the carboxy terminus of the first peptide chain comprise the amino acid sequence Asparagine-Proline-Serine-Xaa-Glycine (SEQ ID NO: 18) where Xaa is any L-amino acid; providing the eukaryotic cell with a second nucleic acid encoding a second peptide chain having the amino acid sequence of SEQ ID NO: 10; and expressing the first peptide chain and the second peptide chain in the eukaryotic cell whereby the expression of the OCT-2 responsive gene is increased relative to a control eukaryotic cell that was not provided with the first and second nucleic acid.

Another aspect of the invention is an in vitro method of increasing the transcription of an OCT-2 responsive gene by a eukaryotic cell comprising the steps of providing a eukaryotic cell comprising an OCT-2 responsive gene; providing the eukaryotic cell with a first nucleic acid comprising a nucleic acid encoding a first peptide chain having an amino acid sequence with at least 90% identity to amino acid residues 1 to 447 of SEQ ID NO: 4 or 6, wherein the five amino acid residues at the carboxy terminus of the first peptide chain comprise the amino acid sequence Asparagine-Proline-Serine-Xaa-Glycine (SEQ ID NO: 18) where Xaa is any L-amino acid; and expressing the first peptide chain in the eukaryotic cell whereby the transcription of the OCT-2 responsive gene is increased relative to a control eukaryotic cell that was not provided with the nucleic acid.

Another aspect of the invention is an in vitro method of increasing the transcription of an OCT-2 responsive gene by a eukaryotic cell comprising the steps of providing a eukaryotic cell comprising an OCT-2 responsive gene; providing the eukaryotic cell with a first nucleic acid comprising a nucleic acid encoding a first peptide chain having an amino acid sequence with at least 90% identity to amino acid residues 1 to 447 of SEQ ID NO: 4 or 6, wherein the five amino acid residues at the carboxy terminus of the first peptide chain comprise the amino acid sequence Asparagine-Proline-Serine-Xaa-Glycine (SEQ ID NO: 18) where Xaa is any L-amino acid; providing the eukaryotic cell with a second nucleic acid encoding a second peptide chain having the amino acid sequence of SEQ ID NO: 10; and expressing the first peptide chain and the second peptide chain in the eukaryotic cell whereby the transcription of the OCT-2 responsive gene is increased relative to a control eukaryotic cell that was not provided with the first and second nucleic acid.

As used herein and in the claims, the singular forms "a," "and," and "the" include plural reference unless the context clearly dictates otherwise. Thus, for example, reference to "a cell" is a reference to one or more cells and includes equivalents thereof known to those skilled in the art.

Unless defined otherwise, all technical and scientific terms used herein have the same meaning as commonly understood by one of ordinary skill in the art to which this invention belongs. Although any compositions and methods similar or equivalent to those described herein can be used in the practice or testing of the invention, exemplary compositions and methods are described herein.

The term "peptide chain" means a molecule that comprises at least two amino acid residues linked by a peptide bond to form a chain. Large peptide chains of more than 50 amino acids may be referred to as "polypeptides" or "proteins." Small peptide chains of less than 50 amino acids may be referred to as "peptides."

The term "nucleic acid" means a molecule that comprises at least two nucleic acid residues linked to form a chain. Such nucleic acid residues may be those found in DNA or RNA.

The term "identity" means the percent identity between two aligned peptide chains. Identity between two peptide chains can be determined by pair-wise amino acid sequence alignment using the default settings of the AlignX module of Vector NTI v.9.0.0 (Invitrogen Corp., Carslbad, CA). AlignX uses the CLUSTALW algorithm to perform pair-wise amino acid sequence alignments.

The term "eukaryotic cell" means a cell in which genetic material is organized into at least one membrane-bound nucleus.

The term "OCT-2 responsive gene" means a nucleic acid that encodes an RNA and responds to OCT-2 activity either directly through the binding of OCT-2 or an OCT-2 homolog to an octameric, consensus 5'-TNATTTGCAT-3' (SEQ ID NO: 15) OCT-2 DNA binding half-site or indirectly to OCT-2 activity. The RNA encoded by an OCT-2 responsive gene may be functional on its own as a small interfering RNA, silencing RNA, or ribozyme. The RNA encoded by an OCT-2 responsive gene may also be translated to produce a peptide chain.

The term "expressing" means the detectable production of a peptide chain encoded by a nucleic acid.

The term "myeloma cell" refers both to cancerous plasma cells obtained, or derived, from an organism with multiple myeloma and to hybridoma cells formed from the fusion of such a cancerous plasma cell with another cell (*e.g*. an antibody producing BALB/c mouse spleen cell or eukaryotic cell stably transfected with a nucleic acid encoding an antibody).

As those skilled in the art will recognize, the peptide chain encoded by the nucleic acid of the invention can be fused at its amino or carboxy termini to a second, heterologous peptide chain. Such heterologous peptide chains may be tags, domains, amino acid linker sequences or other peptide chain types. Examples of peptide chain tags include hexahistidine, flu antigen, and Fc domains. Peptide chain domains may include, for example, transcription activation domains and catalytically active domains such as peroxidases or chloramphenical acetyl transferase as well as other discrete protein domains. Amino acid linker sequences may be sterically unconstrained peptide chains such as those peptide chains that contain multiple glycine, serine, or proline amino acid residues. Those skilled in the art will recognize standard techniques for generating nucleic acids encoding heterologous protein fusions.

Levorotatory-amino acid (L-amino acid) residues include the twenty naturally occurring L-amino acids and naturally occurring post-translational modifications of these L-amino acids such as, for example, selenocysteine and pyrrolysine.

In the isolated nucleic acid disclosed herein, the five amino acid residues at the carboxy terminus of the peptide chain may have the sequence shown in SEQ ID NO: 16 or SEQ ID NO: 17.

In one embodiment the expression vector of the invention comprises a nucleic acid sequence encoding SEQ ID NO: 4. SEQ ID NO: 4 is the amino acid sequence of the *Homo sapiens* OCT-2 Clone #38 variant peptide chain. An exemplary nucleic acid sequence encoding the amino acid sequence of SEQ ID NO: 4 is shown in SEQ ID NO: 3.

In another embodiment the expression vector of the invention comprises a nucleic acid sequence encoding SEQ ID NO: 6. SEQ ID NO: 6 is the amino acid sequence of the *Homo sapiens* OCT-2 Clone #19 variant peptide chain. An exemplary nucleic acid sequence encoding the amino acid sequence of SEQ ID NO: 6 is shown in SEQ ID NO: 5.

Another embodiment of the invention is a cell comprising an expression vector of the invention. Such a cell may be a prokaryotic, eukaryotic, or archaeal cell. It is preferred that such cells be suitable for the expression of peptide chains from the isolated nucleic acids disclosed herein or for the propagation of the isolated nucleic acids disclosed herein.

Another aspect of the invention is an isolated peptide chain comprising an amino acid sequence with at least 90% identity to amino acid residues 1 to 447 of SEQ ID NO: 6, wherein the five amino acid residues at the carboxy terminus of the peptide chain have the amino acid sequence Asparagine-Proline-Serine-Xaa-Glycine (SEQ ID NO: 18) where Xaa is any L-amino acid. As those skilled in the art will recognize, the peptide chain of the invention can be fused to a second, heterologous peptide chain. Such peptide chain fusions can be generated using standard molecular biology techniques to generate amino or carboxy terminal fusions. Alternatively, such peptide chain fusions can be generated by in vitro chemical coupling techniques to fuse peptide chains and generate amino terminal fusions, carboxy terminal fusions, or amino acid side chain fusions.

In the isolated peptide chain disclosed herein the five amino acid residues at the carboxy terminus of the peptide chain may have the amino acid sequence shown in SEQ ID NO: 16 or SEQ ID NO: 17.

In one embodiment the isolated peptide chain of the invention has the amino acid sequence shown in SEQ ID NO: 4.

In another embodiment the isolated peptide chain of the invention has the amino acid sequence shown in SEQ ID NO: 6.

Another aspect of the invention is an in vitro method of increasing the expression of an OCT-2 responsive gene by a eukaryotic cell comprising the steps of providing a eukaryotic cell comprising an OCT-2 responsive gene; providing the eukaryotic cell with a first nucleic acid encoding a first peptide chain having an amino acid sequence with at least 90% identity to amino acid residues 1 to 447 of SEQ ID NO: 6, wherein the five amino acid residues at the carboxy terminus of the first peptide chain have the amino acid sequence Asparagine-Proline-Serine-Xaa-Glycine (SEQ ID NO: 18) where Xaa is any L-amino acid; and expressing the first peptide chain encoded by the first nucleic acid in the eukaryotic cell whereby the expression of the OCT-2 responsive gene is increased relative to a control eukaryotic cell that was not provided with the first nucleic acid.

Eukaryotic cells useful in the method of the invention include mammalian derived cells such as Chinese Hamster Ovary (CHO) cells, and myeloma cells such as SP2/0 cells (American Type Culture Collection (ATCC), Manasas, VA, CRL-1581) and C463A cells. C463A cells and the generation of C463A cells are described in US20030166146A1, which is herein incorporated by reference in its entirety. Such eukaryotic cells may be adapted for growth in chemically defined media lacking animal serum.

The term "OCT-2 responsive gene" is defined above. The RNA encoded by an OCT-2 responsive gene may be functional on its own as a small interfering RNA, silencing RNA, or ribozyme. The RNA encoded by an OCT-2 responsive gene may also be translated to produce a peptide chain. Such peptide chains may be antibody chains, fragments of antibody chains, catalytically active peptide chains, receptor agonist peptide chains, receptor antagonist peptide chains, and other peptide chains with any function that is desirable to express in an cell.

A eukaryotic cell comprises an OCT-2 responsive gene if such a gene is present in the cell. OCT-2 responsive genes may be native genes that have been modified by site specific or random recombination to be OCT-2 responsive genes that are naturally OCT-2 responsive. A native gene can be made OCT-2 responsive by introducing a nucleic acid containing an OCT-2 binding site into the promoter or regulatory region of the native gene. Alternatively, a gene can be made indirectly OCT-2 responsive by introducing a nucleic acid containing a promoter or regulatory region responsive to a transcriptional activator produced as a result of OCT-2 activity. An exogenous OCT-2 responsive gene can also be introduced into a eukaryotic cell. Such an exogenous OCT-2 responsive gene may be, for example, an antibody light or heavy chain gene construct under the control of an OCT-2 responsive promoter such as an immunoglobulin promoter. Site specific, targed recombination can also be used to place an exogenous gene under the control of an endogenous OCT-2 responsive regulatory region or promoter. Standard molecular biology, recombinant gene technology techniques and cell culture techniques well known to those skilled in the art can be used for the construction of OCT-2 responsive genes either *in vitro* or in vivo and for the identification of eukaryotic cells comprising an OCT-2 responsive gene.

A nucleic acid may be provided to eukaryotic cells in the method of the invention by well-known techniques such as cell fusion, electroporation, lipofection, viral infection, and calcium phosphate precipitation based techniques. Those skilled in the art will recognize other techniques for providing a nucleic acid to a eukaryotic cell.

Expression of an OCT-2 responsive gene is increased relative to a control eukaryotic cell when the level or activity of the peptide chain encoded by the OCT-2 responsive gene is increased relative to the control eukaryotic cell. Peptide chain levels may be measured by any means known in the art such as, for example, SDS-PAGE. Peptide chain activity levels can be measured using activity assays specific to the activity of the peptide chain. For example, antibody peptide chain expression may be measured by SDS-PAGE, and the antigen binding activity of an antibody may be measured using standard ELISA techniques well known in the art. Peptide chain levels or activity may be numerically expressed using any appropriate units and normalized if necessary. Normalization can be accomplished by using the level of a second peptide chain, the number of cells in a sample, or on the basis of elapsed time, for example.

In one embodiment of the method of the invention the first five amino acid residues at the carboxy terminus of the first peptide chain have the sequence shown in SEQ ID NO: 16 or SEQ ID NO: 17.

In another embodiment of the method of the invention the first peptide chain has the amino acid sequence shown in SEQ ID NO: 4.

In another embodiment of the method of the invention the first peptide chain has the amino acid sequence shown in SEQ ID NO: 6.

In another embodiment of the method of the invention the eukaryotic cell is a myeloma cell. Examples of myeloma cell lines useful in the.methods of the invention include the SP2/0, NSO (European Collection of Cell Cultures (ECACC), Salisbury, Wiltshire, UK, ECACC No. 85110503), FO (ATCC CRL-1646), and Ag653 (ATCC CRL-1580) cell lines which were obtained from mice. An example of a myeloma cell line obtained from humans and useful in the methods of the invention is the U266 cell line (ATTC CRL-TIB-196). The C463A myeloma cell line is also useful in the methods of the invention and is an example of an SP2/0 derived cell line capable of growing in chemically defined media. Those skilled in the art will recognize other myeloma cell lines.

In another embodiment of the method of the invention the eukaryotic cell is selected from the group consisting of SP2/0, C463A, and CHO cells. Each of these cell types have the common properties of being suitable for in vitro culture and having the ability to express peptide chains at high levels.

Another aspect of the invention is an in vitro method of increasing the expression of an OCT-2 responsive gene by a eukaryotic cell comprising the steps of providing a eukaryotic cell comprising an OCT-2 responsive gene; providing the eukaryotic cell with a first nucleic acid encoding a first peptide chain having an amino acid sequence with at least 90% identity to amino acid residues 1 to 447 of SEQ ID NO: 6, wherein the five amino acid residues at the carboxy terminus of the first peptide chain have the amino acid sequence of the Asparagine-Proline-Serine-Xaa-Glycine (SEQ ID NO: 18) where Xaa is any L-amino acid; providing the eukaryotic cell with a second nucleic acid encoding a second peptide chain having the amino acid sequence shown in SEQ ID NO: 8 or SEQ ID NO: 10; and expressing the first peptide chain and the second peptide chain in the eukaryotic cell whereby the expression of the OCT-2 responsive gene is increased relative to a control eukaryotic cell that was not provided with the first and second nucleic acid. SEQ ID NO 8 is the amino acid sequence of the *Homo sapiens* OBF-1 peptide chain. An exemplary nucleic acid sequence encoding the amino acid sequence of SEQ ID NO: 8 is shown in SEQ ID NO: 7. SEQ ID NO 10 is the amino acid sequence of the *Mus musculus* OBF-1 peptide chain. An exemplary nucleic acid sequence encoding the amino acid sequence of SEQ ID NO: 10 is shown in SEQ ID NO: 9.

In one embodiment of the method of the invention the first five amino acid residues at the carboxy terminus of the first peptide chain have the sequence shown in SEQ ID NO: 16 or SEQ ID NO: 17.

In another embodiment of the method of the invention, the OCT-2 responsive gene can be an antibody gene, such as a heavy or light chain gene.

In another embodiment of the method of the invention the first peptide chain has the amino acid sequence shown in SEQ ID NO: 4.

In another embodiment of the method of the invention the first peptide chain has the amino acid sequence shown in SEQ ID NO: 6.

In another embodiment of the method of the invention the eukaryotic cell is a myeloma cell.

In another embodiment of the method of the invention the eukaryotic cell is selected from the group consisting of SP2/0, C463A, and CHO cells.

Another aspect of the invention is an in vitro method of increasing the expression of an OCT-2 responsive antibody gene by an eukaryotic cell comprising the steps of providing a eukaryotic cell comprising an OCT-2 responsive antibody gene; providing the eukaryotic cell with a first nucleic acid encoding a first peptide chain having the sequence shown in SEQ ID NO: 4 or SEQ ID NO: 6; and expressing the first peptide chain encoded by the first nucleic acid in the eukaryotic cell whereby the expression of the OCT-2 responsive antibody gene is increased relative to a control eukaryotic cell that was not provided with the first nucleic acid.

In one embodiment of the method of the invention the eukaryotic cell is selected from the group consisting of SP2/0, C463A, and CHO cells.

Another aspect of the invention is an in vitro method of increasing the expression of an OCT-2 responsive antibody gene by a eukaryotic cell comprising the steps of providing a eukaryotic cell comprising an OCT-2 responsive gene; providing the eukaryotic cell with a first nucleic acid encoding a first peptide chain having the sequence shown in SEQ ID NO: 4 or SEQ ID NO: 6; providing the eukaryotic cell with a second nucleic acid encoding a second peptide chain having the amino sequence shown in SEQ ID NO: 10; and expressing the first peptide chain and the second peptide chain in the eukaryotic cell whereby the expression of the OCT-2 responsive antibody gene is increased relative to a control eukaryotic cell that was not provided with the first and second nucleic acid.

In one embodiment of the method of the invention the eukaryotic cell is selected from the group consisting of SP2/0, C463A, and CHO cells.

Another aspect of the invention is an in vitro method of increasing the transcription of an OCT-2 responsive gene by a eukaryotic cell comprising the steps of providing a eukaryotic cell comprising an OCT-2 responsive gene; providing the eukaryotic cell with a first nucleic acid comprising a nucleic acid encoding a first peptide chain having an amino acid sequence with at least 90% identity to amino acid residues 1 to 447 of SEQ ID NO: 4 or 6, wherein the five amino acid residues at the carboxy terminus of the first peptide chain have the amino acid sequence Asparagine-Proline-Serine-Xaa-Glycine (SEQ ID NO: 18) where Xaa is any L-amino acid; and expressing the first peptide in the eukaryotic cell whereby the transcription of the OCT-2 responsive gene is increased relative to a control eukaryotic cell that was not provided with the nucleic acid.

Transcription of an OCT-2 responsive gene is increased relative to a control eukaryotic cell when the level or activity of the RNA transcript encoded by the OCT-2 responsive gene is increased relative to the control eukaryotic cell. RNA transcript levels may be measured by any means known in the art such as, for example RT-PCR and Northern blots. RNA transcript activity levels can be measured using ribozyme activity assays, silencing RNA assays, or antisense-RNA assays specific to the activity of the RNA transcript for example. RNA transcript levels or activity may be numerically expressed using any appropriate units and normalized if necessary. Normalization can be accomplished by using the level of a second RNA transcript, the number of cells in a sample, or on the basis of elapsed time, for example.

Another aspect of the invention is an in vitro method of increasing the transcription of an OCT-2 responsive gene by a eukaryotic cell comprising the steps of providing a eukaryotic cell comprising an OCT-2 responsive gene; providing the eukaryotic cell with a first nucleic acid comprising a nucleic accid encoding a first peptide chain having an amino acid sequence with at least 90% identity to amino acid residues 1 to 447 of SEQ ID NO: 4 or 6, wherein the five amino acid residues at the carboxy terminus of the first peptide chain have the amino acid sequence Asparagine-Proline-Serine-Xaa-Glycine (SEQ ID NO: 18) where Xaa is any L-amino acid; providing the eukaryotic cell with a second nucleic acid encoding a second peptide chain having the amino acid sequence of SEQ ID NO: 10; and expressing the first peptide chain and the second peptide chain in the eukaryotic cell whereby the transcription of the OCT-2 responsive gene is increased relative to a control eukaryotic cell that was not provided with the first and second nucleic acid.

The present invention is further described with reference to the following examples. These examples are merely to illustrate aspects of the present invention and are not intended as limitations of this invention.

### Example 1

### Isolation of cDNA Encoding Homo sapiens OCT-2 Variant Proteins

Two cDNAs encoding the *Homo sapiens* OCT-2 Clone #38 (SEQ ID NO: 4) and *Homo sapiens* OCT-2 Clone #19 (SEQ ID NO: 6) variant proteins were isolated and sequenced. The cDNA (SEQ ID NO: 3) encoding the *Homo sapiens* OCT-2 Clone #38 variant protein (SEQ ID NO: 4) and the cDNA (SEQ ID NO: 5) encoding the *Homo sapiens* OCT-2 Clone #19 variant protein (SEQ ID NO: 6) were isolated using standard polymerase chain reaction (PCR) techniques from a *Homo sapiens* cDNA library (Stratagene Inc., La Jolla, CA).

The nucleic acid sequences of the forward primer and reverse primer used in the PCR amplification of SEQ ID NO: 4 and SEQ ID NO: 6 encoding these *Homo sapiens* OCT-2 protein variants are shown in SEQ ID NO: 11 (forward primer) and SEQ ID NO: 12 (reverse primer). These primers were designed using the nucleic acid sequence described in Accession M36653. Accession M36653 contains two open reading frames, designated CDS 1 and CDS 2, and encodes a *Homo sapiens* OCT-2 protein. This *Homo sapiens* OCT-2 protein is predicted to be encoded by CDS 1. The forward (SEQ ID NO: 11) and reverse (SEQ ID NO: 12) primers are specific to the 5' untranslated region (UTRs) flanking CDS 1 in Accession M36653 and a sequence located 3' to CDS 1 of the nucleic acid sequence described by Accession M36653. PCR using these primers amplifies any library nucleic acid sequences located between the binding sites of these two primers. Amplified DNA fragments resulting from PCR using these primers were isolated, cloned into the pCDNA3.1 expression vector (Invitrogen Inc., Carlsbad, CA), and sequenced using standard molecular biology techniques.

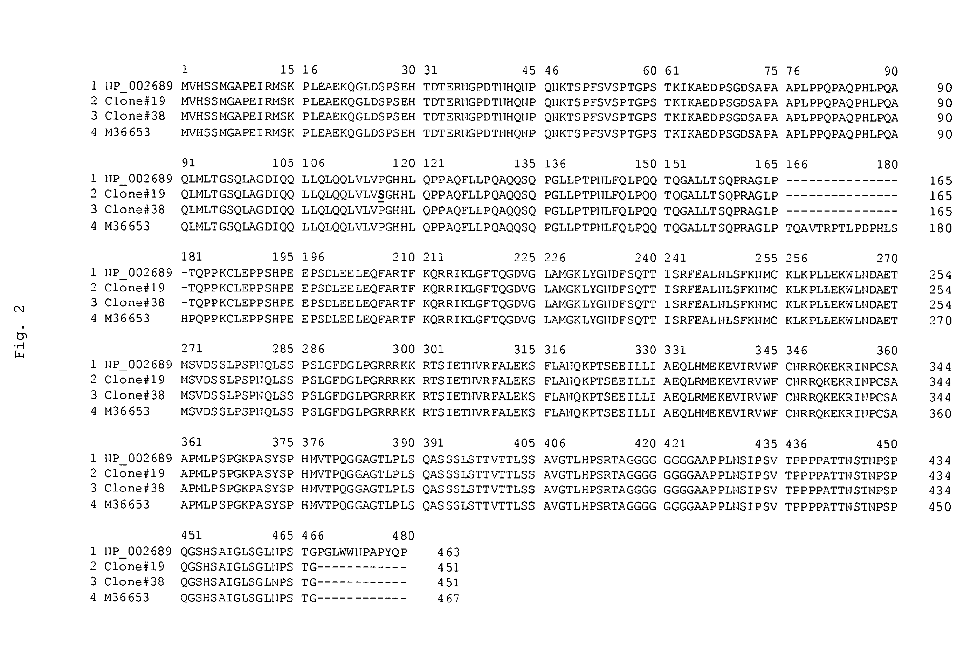

Sequencing, conceptual translations, and multiple sequence alignment analysis (Fig. 2) revealed that two unique nucleic acid sequences encoding the *Homo sapiens* OCT-2 Clone #38 protein and *Homo sapiens* OCT-2 Clone #19 protein had been isolated. This analysis showed these two clones encoded *Homo sapiens* OCT-2 variant proteins that are different than the wild-type, archetypical *Homo sapiens* OCT-2 protein (SEQ ID NO: 1) described by Accession NP_002689 and the *Homo sapiens* OCT-2 protein (SEQ ID NO: 14) predicted to be encoded by CDS 1 (SEQ ID NO: 13) of Accession M36653. As seen in the multiple protein sequence alignments of Fig. 2 the *Homo sapiens* OCT-2 Clone #38 protein sequence (SEQ ID NO: 4) and *Homo sapiens* OCT-2 Clone #19 protein sequence (SEQ ID NO: 6) both lack twelve carboxy-terminal amino acid residues (SEQ ID NO: 19) found in the wild-type, archetypal *Homo sapiens* OCT-2 protein sequence described by Accession NP_002689 (SEQ ID NO: 2). Further, as seen in the multiple protein sequence alignments of Fig. 2 the *Homo sapiens* OCT-2 Clone #38 protein sequence (SEQ ID NO: 4) and *Homo sapiens* OCT-2 Clone #19 protein sequence (SEQ ID NO: 6) both lack an 11 amino acid residue sequence found at position 166 to 181 of the *Homo sapiens* OCT-2 protein predicted to be encoded by CDS 1 of Accession M36653 (SEQ ID NO: 14). Lastly, multiple protein sequence alignment (Fig. 2) also revealed that the *Homo sapiens* OCT-2 Clone #19 protein sequence (SEQ ID NO: 6) has an serine (S) amino acid residue at position 116, instead of the proline (P) residue found at position 116 in the wild-type, archetypical *Homo sapiens* OCT-2 (SEQ ID NO: 2), Clone #38 protein sequences (SEQ ID NO: 4), and the *Homo sapiens* OCT-2 protein predicted to be encoded by CDS 1 of Accession M36653 (SEQ ID NO: 14). Multiple sequence alignment analysis was performed using the CLUSTALW algorithm and the CLUSTALW default settings. These results demonstrate that two new *Homo sapiens* OCT-2 protein variants had been isolated and identified.

### Example 2

### Overexpression of OCT-2 Variant Protein and OBF-1 Increases Antibody Gene Transcript and Expression Levels

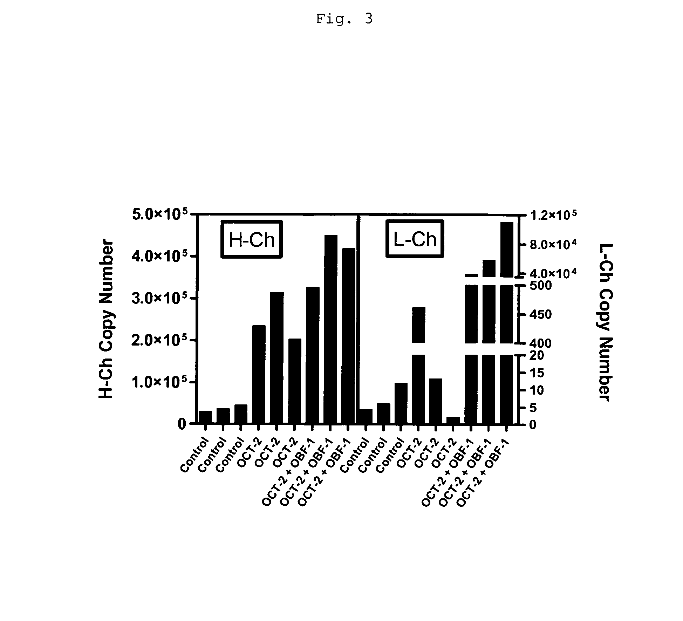

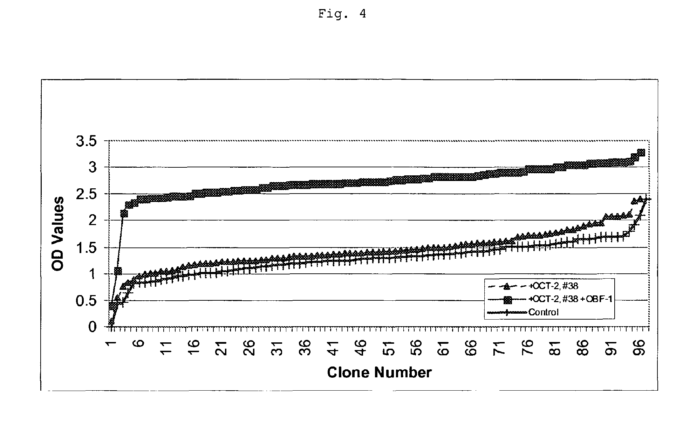

Stable overexpression of *Homo sapiens* OCT-2 Clone #38 variant protein alone (SEQ ID NO: 4), and in combination with overexpression of *Mus musculus* OBF-1 (SEQ ID NO: 10) increased recombinant antibody heavy and light chain gene transcript (Fig. 3) and expression levels (Fig. 4) in C463A cells.

Control C463A cells for this experiment were stably cotransfected using standard methods, with a heavy chain expression vector and a light chain expression vector. OCT-2 transfected cells were stably cotransfected with the heavy chain expression vector, the light chain expression vector, and a pcDNA3.1/hOCT-2 vector encoding *Homo sapiens* OCT-2 Clone #38 (SEQ ID NO: 4). OCT-2 and OBF-1 transfected cells were stably contransfected with the the heavy chain expression vector, the light chain expression vector, pcDNA3.1/hOCT-2 and pcDNA3.1/mOBF-1 vector encoding *Mus musculus* OBF-1 (SEQ ID NO: 10).

C463A cells were derived from *Mus musculus* SP2/0 myeloma cells and were adapted for growth in chemically defined culture medium. The heavy and light chain expression vectors encode the heavy and light chains of a fully human, tumor necrosis factor-alpha (TNF-alpha) specific, monoclonal antibody. An immunoglobulin promoter driving heavy and light chain expression by the heavy chain expression vector and the light chain expression vectors contains an octameric, consensus (5'-TNATTTGCAT-3'; SEQ ID NO: 15) OCT-2 DNA binding half-site. This OCT-2 DNA site makes heavy and light chain gene transcription from the heavy chain expression vector and the light chain expression vector responsive to OCT-2 activity. The heavy chain expression vector, light chain expression vector, pcDNA3.1/hOCT-2 and pcDNA3.1/mOBF-1 vectors each constitutively express the various proteins they encode.

Transfections were performed by electroporation of approximately 1x10⁷ C463A cells using standard methods. Control C463A cells were stably transfected by electroporation with 4 µg of heavy chain expression vector, 4 µg light chain expression vector, and 2 µg pcDNA3.1. OCT-2 C463A cells were stably transfected with 4 µg of heavy chain expression vector, 4 µg of light chain expression vector, and 2 µg pcDNA3.1/hOCT-2. OCT-2 C463A cells were stably transfected with 4 µg of heavy chain expression vector, 4 µg of light chain expression vector, 2 µg pcDNA3.1/hOCT-2 and 2 µg pcDNA3.1/mOBF-1. MHX (mycophenolic acid, hypoxanthine, and xanthine) and standard methods were used to select cells stably transfected with the heavy chain expression vector and light chain expression vector. G418 and standard methods were used to select cells stably transfected with pcDNA3.1/hOCT-2 and pcDNA3.1/mOBF-1 cells. ELISA assays were then performed using standard methods to identify clones with the highest expression of the fully human, recombinant, tumor necrosis factor-alpha (TNF-alpha) specific, monoclonal antibody encoded by the heavy chain expression vector and light chain expression vector (Fig. 4).

Stable expression of the *Homo sapiens* OCT-2 Clone #38 variant protein alone increased recombinant antibody gene transcript and expression levels relative to control cells (Fig. 3 and Fig. 4). Stable co-expression of the *Homo sapiens* OCT-2 Clone #38 (SEQ ID NO: 4) variant protein and *Mus musculus* OBF-1 protein (SEQ ID NO: 10) further increased recombinant antibody expression relative to control cells and cells expressing OCT-2 Clone #38 variant protein (SEQ ID NO: 4) alone (Fig. 3 and Fig. 4). Together these results indicate that the *Homo sapiens* OCT-2 Clone #38 variant protein is biologically active, capable of binding OCT-2 DNA binding sites to enhance gene activation and is capable of interacting with the *Mus musculus* OBF-1 protein to activate OCT-2 responsive gene expression.

## Claims

1. An expression vector comprising a nucleic acid encoding a peptide chain comprising an amino acid sequence, wherein the nucleic acid encodes a peptide chain having:
(i) the amino acid sequence shown in SEQ ID NO: 4; or
(ii) the amino acid sequence shown in SEQ ID NO: 6.

2. An isolated peptide chain comprising an amino acid sequence , wherein the peptide chain has:
(i) the amino acid sequence shown in SEQ ID NO: 4; or
(ii) the amino acid sequence shown in SEQ ID NO: 6.

3. A cell comprising the expression vector of claim 1.

4. An in vitro method of increasing the expression of an OCT-2 responsive gene by a eukaryotic cell comprising the steps of:
a) providing a eukaryotic cell comprising an OCT-2 responsive gene;
b) providing the eukaryotic cell with a first nucleic acid comprising a nucleic acid encoding a first peptide chain having an amino acid sequence with at least 90% identity to amino acid residues 1 to 447 of SEQ ID NO: 6; wherein the five amino acid residues at the carboxy terminus of the first peptide chain have the amino acid sequence Asparagine-Proline-Serine-Xaa-Glycine (SEQ ID NO: 18) where Xaa is any L-amino acid; and
c) expressing the first peptide chain encoded by the first nucleic acid in the eukaryotic cell whereby the expression of the OCT-2 responsive gene is increased relative to a control eukaryotic cell that was not provided with the first nucleic acid.

5. An in vitro method of increasing the expression of an OCT-2 responsive gene by a eukaryotic cell comprising the steps of:
a) providing a eukaryotic cell comprising an OCT-2 responsive gene;
b) providing the eukaryotic cell with a first nucleic acid
comprising a nucleic acid encoding a first peptide chain having an amino acid sequence with at least 90% identity to amino acid residues 1 to 447 of SEQ ID NO: 6, wherein the five amino acid residues at the carboxy terminus of the first peptide chain have the amino acid sequence Asparagine-Proline-Serine-Xaa-Glycine (SEQ ID NO: 18) where Xaa is any L-amino acid;
c) providing the eukaryotic cell with a second nucleic acid encoding a second peptide chain having the amino acid sequence shown in SEQ ID NO: 8 or SEQ ID NO: 10; and
d) expressing the first peptide chain and the second peptide chain in the eukaryotic cell whereby the expression of the OCT-2 responsive gene is increased relative to a control eukaryotic cell that was not provided with the first and second nucleic acid.

6. The in vitro method of claim 4 or 5 wherein the first five amino acid residues at the carboxy terminus of the first peptide chain have the sequence shown in SEQ ID NO: 16 and SEQ ID NO: 17.

7. The in vitro method of claim 4, 5 or 6 wherein the first peptide chain has:
(i) the amino acid sequence shown in SEQ ID NO: 4; or
(ii) the amino acid sequence shown in SEQ ID NO: 6.

8. The in vitro method of any one of claims 4-7 wherein the eukaryotic cell is:
(i) a myeloma cell.
(ii) selected from the group consisting of SP2/0, C463A, and CHO cells.

9. The in vitro method of any one of claims 4-8 wherein the OCT-2 responsive gene is an antibody gene.

10. An in vitro method of increasing the transcription of an OCT-2 responsive gene by a eukaryotic cell comprising the steps of:
a) providing a eukaryotic cell comprising an OCT-2 responsive gene;
b) providing the eukaryotic cell with a first nucleic acid comprising a nucleic acid encoding a first peptide chain having an amino acid sequence with at least 90% identity to amino acid residues 1 to 447 of SEQ ID NO: 4 or 6, wherein the five amino acid residues at the carboxy terminus of the first peptide chain have the amino acid sequence Asparagine-Proline-Serine-Xaa-Glycine (SEQ ID NO: 18) where Xaa is any L-amino acid; and
c) expressing the first peptide chain in the eukaryotic cell whereby the transcription of the OCT-2 responsive gene is increased relative to a control eukaryotic cell that was not provided with the nucleic acid.
/

11. An in vitro method of increasing the transcription of an OCT-2 responsive gene by a eukaryotic cell comprising the steps of:
a) providing a eukaryotic cell comprising an OCT-2 responsive gene;
b) providing the eukaryotic cell with a first nucleic acid comprising a nucleic acid encoding a first peptide chain having an amino acid sequence with at least 90% identity to amino acid residues 1 to 447 of SEQ ID NO: 4 or 6, wherein the five amino acid residues at the carboxy terminus of the first peptide chain comprise the amino acid sequence Asparargine-Proline-Serine-Xaa-Glycine (SEQ ID NO: 18) where Xaa is any L-amino acid;
c) providing the eukaryotic cell with a second nucleic acid encoding a second peptide chain having the amino acid sequence of SEQ ID NO: 10; and
d) expressing the first peptide chain and the second peptide chain in the eukaryotic cell whereby the transcription of the OCT-2 responsive gene is increased relative to a control eukaryotic cell that was not provided with the first and second nucleic acid.

12. The in vitro method of claim 10 or 11 wherein the eukaryotic cell is selected from the group consisting of SP2/0, C463A, and CHO cells.

13. The in vitro method of claim 12 wherein the eukaryotic cell is C463a.

## Patentansprüche

1. Expressionsvektor, umfassend eine Nukleinsäure, die eine Peptidkette codiert, die eine Aminosäuresequenz umfasst, wobei die Nukleinsäure eine Peptidkette mit
(i) der in SEQ ID NR: 4 dargestellten Aminosäuresequenz oder
(ii) der in SEQ ID NR: 6 dargestellten Aminosäuresequenz
codiert.

2. Isolierte Peptidkette, die eine Aminosäuresequenz umfasst, wobei die Peptidkette
(i) die in SEQ ID NR: 4 dargestellte Aminosäuresequenz oder
(ii) die in SEQ ID NR: 6 dargestellte Aminosäuresequenz besitzt.

3. Zelle, die den Expressionsvektor nach Anspruch 1 umfasst.

4. In vitro-Verfahren zur Erhöhung der Expression eines OCT-2-responsiven Gens durch eine eukaryotische Zelle, das folgende Schritte umfasst:
(a) Bereitstellen einer eukaryotischen Zelle, die ein OCT-2-responsives Gen umfasst,
(b) Versehen der eukaryotischen Zelle mit einer ersten Nukleinsäure, die eine Nukleinsäure umfasst, die eine erste Peptidkette mit einer Aminosäuresequenz mit mindestens 90% Identität zu den Aminosäureresten 1 bis 447 der SEQ ID NR: 6 codiert, wobei die fünf Aminosäurereste am Carboxyterminus der ersten Peptidkette die Aminosäuresequenz Asparagin-Prolin-Serin-Xaa-Glycin (SEQ ID NR: 18), wobei Xaa eine beliebige L-Aminosäure ist, besitzen,
und
(c) Exprimieren der von der ersten Nukleinsäure codierten ersten Peptidkette in der eukaryotischen Zelle, wodurch die Expression des OCT-2-responsiven Gens im Vergleich zu einer eukaryotischen Kontrollzelle, die nicht mit der ersten Nukleinsäure versehen wurde, erhöht ist.

5. In vitro-Verfahren zur Erhöhung der Expression eines OCT-2-responsiven Gens durch eine eukaryotische Zelle, das folgende Schritte umfasst:
(a) Bereitstellen einer eukaryotischen Zelle, die ein OCT-2-responsives Gen umfasst,
(b) Versehen der eukaryotischen Zelle mit einer ersten Nukleinsäure,
die eine Nukleinsäure umfasst, die eine erste Peptidkette mit einer Aminosäuresequenz mit mindestens 90% Identität zu den Aminosäureresten 1 bis 447 der SEQ ID NR: 6 codiert, wobei die fünf Aminosäurereste am Carboxyterminus der ersten Peptidkette die Aminosäuresequenz Asparagin-Prolin-Serin-Xaa-Glycin (SEQ ID NR: 18), wobei Xaa eine beliebige L-Aminosäure ist, besitzen,
(c) Versehen der eukaryotischen Zelle mit einer zweiten Nukleinsäure, die eine zweite Peptidkette mit der in SEQ ID NR: 8 oder SEQ ID NR: 10 dargestellten Aminosäuresequenz codiert, und
(d) Exprimieren der ersten Peptidkette und der zweiten Peptidkette in der eukaryotischen Zelle, wodurch die Expression des OCT-2-responsiven Gens im Vergleich zu einer eukaryotischen Kontrollzelle, die nicht mit der ersten und der zweiten Nukleinsäure versehen wurde, erhöht ist.

6. In vitro-Verfahren nach Anspruch 4 oder 5, wobei die fünf Aminosäurereste am Carboxyterminus der ersten Peptidkette die in SEQ ID NR: 16 und SEQ ID NR: 17 dargestellte Sequenz besitzen.

7. In vitro-Verfahren nach Anspruch 4, 5 oder 6, wobei die erste Peptidkette
(i) die in SEQ ID NR: 4 dargestellte Aminosäuresequenz oder
(ii) die in SEQ ID NR: 6 dargestellte Aminosäuresequenz
besitzt.

8. In vitro-Verfahren nach einem der Ansprüche 4-7, wobei die eukaryotische Zelle:
(i) eine Myelomzelle ist,
(ii) aus der Gruppe ausgewählt wird, die aus SP2/0-, C463A- und CHO-Zellen besteht.

9. In vitro-Verfahren nach einem der Ansprüche 4-8, wobei das OCT-2-responsive Gen ein Antikörpergen ist.

10. In vitro-Verfahren zur Erhöhung der Transkription eines OCT-2-responsiven Gens durch eine eukaryotische Zelle, das folgende Schritte umfasst:
(a) Bereitstellen einer eukaryotischen Zelle, die ein OCT-2-responsives Gen umfasst,
(b) Versehen der eukaryotischen Zelle mit einer ersten Nukleinsäure, die eine Nukleinsäure umfasst, die eine erste Peptidkette mit einer Aminosäuresequenz mit mindestens 90% Identität zu den Aminosäureresten 1 bis 447 der SEQ ID NR: 4 oder 6 codiert, wobei die fünf Aminosäurereste am Carboxyterminus der ersten Peptidkette die Aminosäuresequenz Asparagin-Prolin-Serin-Xaa-Glycin (SEQ ID NR: 18), wobei Xaa eine beliebige L-Aminosäure ist, besitzen, und
(c) Exprimieren der ersten Peptidkette in der eukaryotischen Zelle, wodurch die Transkription des OCT-2-responsiven Gens im Vergleich zu einer eukaryotischen Kontrollzelle, die nicht mit der Nukleinsäure versehen wurde, erhöht ist.

11. In vitro-Verfahren zur Erhöhung der Transkription eines OCT-2-responsiven Gens durch eine eukaryotische Zelle, das folgende Schritte umfasst:
(a) Bereitstellen einer eukaryotischen Zelle, die ein OCT-2-responsives Gen umfasst,
(b) Versehen der eukaryotischen Zelle mit einer ersten Nukleinsäure, die eine Nukleinsäure umfasst, die eine erste Peptidkette mit einer Aminosäuresequenz mit mindestens 90% Identität zu den Aminosäureresten 1 bis 447 der SEQ ID NR: 4 oder 6 codiert, wobei die fünf Aminosäurereste am Carboxyterminus der ersten Peptidkette die Aminosäuresequenz Asparagin-Prolin-Serin-Xaa-Glycin (SEQ ID NR: 18), wobei Xaa eine beliebige L-Aminosäure ist, besitzen,
(c) Versehen der eukaryotischen Zelle mit einer zweiten Nukleinsäure, die eine zweite Peptidkette mit der Aminosäuresequenz SEQ ID NR: 10 codiert, und
(d) Exprimieren der ersten Peptidkette und der zweiten Peptidkette in der eukaryotischen Zelle, wodurch die Transkription des OCT-2-responsiven Gens im Vergleich zu einer eukaryotischen Kontrollzelle, die nicht mit der ersten und der zweiten Nukleinsäure versehen wurde, erhöht ist.

12. In vitro-Verfahren nach Anspruch 10 oder 11, wobei die eukaryotische Zelle aus der Gruppe ausgewählt wird, die aus SP2/0-, C463A- und CHO-Zellen besteht.

13. In vitro-Verfahren nach Anspruch 12, wobei die eukaryotische Zelle C463A ist.

## Revendications

1. Vecteur d'expression comprenant un acide nucléique codant pour une chaîne peptidique, qui comprend une séquence d'acides aminés, dans lequel l'acide nucléique code pour une chaîne peptidique ayant :
(i) la séquence d'acides aminés présentée dans la SEQ ID n° : 4 ; ou
(ii) la séquence d'acides aminés présentée dans la SEQ ID n° : 6.

2. Chaîne peptidique isolée comprenant une séquence d'acides aminés, dans laquelle la chaîne peptidique a :
(i) la séquence d'acides aminés présentée dans la SEQ ID n° : 4 ; ou
(ii) la séquence d'acides aminés présentée dans la SEQ ID n° : 6.

3. Cellule comprenant le vecteur d'expression selon la revendication 1.

4. Procédé *in vitro* d'augmentation de l'expression d'un gène sensible à l'OCT-2 par une cellule eucaryote, comprenant les étapes consistant à :
a) fournir une cellule eucaryote comprenant un gène sensible à l'OCT-2 ;
b) pourvoir la cellule eucaryote d'un premier acide nucléique, comprenant un acide nucléique qui code pour une première chaîne peptidique ayant une séquence d'acides aminés avec une identité d'au moins 90% avec les résidus d'acides aminés 1 à 447 de la SEQ ID n° : 6, dans laquelle les cinq résidus d'acides aminés à l'extrémité carboxy-terminale de la première chaîne peptidique ont la séquence d'acides aminés Asparagine-Proline-Sérine-Xaa-Glycine (SEQ ID n° : 18), dans laquelle Xaa est un acide aminé L quelconque ; et
c) exprimer la première chaîne peptidique codée par le premier acide nucléique dans la cellule eucaryote, moyennant quoi l'expression du gène sensible à l'OCT-2 est accrue par rapport à une cellule eucaryote témoin qui n'a pas été pourvue du premier acide nucléique.

5. Procédé *in vitro* d'augmentation de l'expression d'un gène sensible à l'OCT-2 par une cellule eucaryote, comprenant les étapes consistant à :
a) fournir une cellule eucaryote comprenant un gène sensible à l'OCT-2 ;
b) pourvoir la cellule eucaryote d'un premier acide nucléique
comprenant un acide nucléique qui code pour une première chaîne peptidique ayant une séquence d'acides aminés avec une identité d'au moins 90% avec les résidus d'acides aminés 1 à 447 de la SEQ ID n° : 6, dans laquelle les cinq résidus d'acides aminés à l'extrémité carboxy-terminale de la première chaîne peptidique ont la séquence d'acides aminés Asparagine-Proline-Sérine-Xaa-Glycine (SEQ ID n° : 18), dans laquelle Xaa est un acide aminé L quelconque ;
c) pourvoir la cellule eucaryote d'un deuxième acide nucléique codant pour une deuxième chaîne peptidique, qui a la séquence d'acides aminés présentée dans la SEQ ID n° : 8 ou la SEQ ID n° : 10 ; et
d) exprimer la première chaîne peptidique ainsi que la deuxième chaîne peptidique dans la cellule eucaryote, moyennant quoi l'expression du gène sensible à l'OCT-2 est accrue par rapport à une cellule eucaryote témoin qui n'a pas été pourvue des premier et deuxième acides nucléiques.

6. Procédé *in vitro* selon la revendication 4 ou la 5, dans lequel les cinq premiers résidus d'acides aminés à l'extrémité carboxy-terminale de la première chaîne peptidique ont la séquence présentée dans les SEQ ID n° : 16 et SEQ ID n° : 17.

7. Procédé *in vitro* selon la revendication 4, la 5 ou la 6, dans lequel la première chaîne peptidique a :
(i) la séquence d'acides aminés présentée dans la SEQ ID n° : 4 ; ou
(ii) la séquence d'acides aminés présentée dans la SEQ ID n° : 6.

8. Procédé *in vitro* selon l'une quelconque des revendications 4 à 7, dans lequel la cellule eucaryote est :
(i) une cellule de myléome.
(ii) choisie dans le groupe constitué par les cellules SP2/0, C463A et CHO.

9. Procédé *in vitro* selon l'une quelconque des revendications 4 à 8, dans lequel le gène sensible à l'OCT-2 est un gène d'anticorps.

10. Procédé *in vitro* d'augmentation de la transcription d'un gène sensible à l'OCT-2 par une cellule eucaryote, comprenant les étapes consistant à :
a) fournir une cellule eucaryote comprenant un gène sensible à l'OCT-2 ;
b) pourvoir la cellule eucaryote d'un premier acide nucléique, comprenant un acide nucléique qui code pour une première chaîne peptidique ayant une séquence d'acides aminés avec une identité d'au moins 90% avec les résidus d'acides aminés 1 à 447 de la SEQ ID n° : 4 ou de la 6, dans laquelle les cinq résidus d'acides aminés à l'extrémité carboxy-terminale de la première chaîne peptidique ont la séquence d'acides aminés Asparagine-Proline-Sérine-Xaa-Glycine (SEQ ID n° : 18), dans laquelle Xaa est un acide aminé L quelconque ; et
c) exprimer la première chaîne peptidique dans la cellule eucaryote, moyennant quoi la transcription du gène sensible à l'OCT-2 est accrue par rapport à une cellule eucaryote témoin qui n'a pas été pourvue du premier acide nucléique.

11. Procédé *in vitro* d'augmentation de la transcription d'un gène sensible à l'OCT-2 par une cellule eucaryote, comprenant les étapes consistant à :
a) fournir une cellule eucaryote comprenant un gène sensible à l'OCT-2 ;
b) pourvoir la cellule eucaryote d'un premier acide nucléique comprenant un acide nucléique qui code pour une première chaîne peptidique ayant une séquence d'acides aminés avec une identité d'au moins 90% avec les résidus d'acides aminés 1 à 447 de la SEQ ID n° : 4 ou de la 6, dans laquelle les cinq résidus d'acides aminés à l'extrémité carboxy-terminale de la première chaîne peptidique comprennent la séquence d'acides aminés Asparagine-Proline-Sérine-Xaa-Glycine (SEQ ID n° : 18), dans laquelle Xaa est un acide aminé L quelconque ;
c) pourvoir la cellule eucaryote d'un deuxième acide nucléique codant pour une deuxième chaîne peptidique, qui a la séquence d'acides aminés de SEQ ID n° : 10 ; et
d) exprimer la première chaîne peptidique ainsi que la deuxième chaîne peptidique dans la cellule eucaryote, moyennant quoi l'expression du gène sensible à l'OCT-2 est accrue par rapport à une cellule eucaryote témoin qui n'a pas été pourvue des premier et deuxième acides nucléiques.

12. Procédé *in vitro* selon la revendication 10 ou la 11, dans lequel la cellule eucaryote est choisie dans le groupe constitué par les cellules SP2/0, C463A et CHO.

13. Procédé *in vitro* selon la revendication 12, dans lequel la cellule eucaryote est une C463a.
